# EUROPEAN PATENT APPLICATION

(11) **EP 3 733 755 A1**
(43) Date of publication of application: **04.11.2020**
(21) Application number: 20172421.8
(22) Date of filing: 30.04.2020
(51) Int. Cl.: C08L 5/08, A61K 47/36, A61L 27/20, C08B 37/08

(54) **HOMOGENEOUS HYDROGELS FROM CHITOSAN OLIGOSACCHARIDE DERIVATIVES AND APPLICATIONS THEREOF**

(30) Priority: 30.04.2019 IT 201900006448
(71) Applicant: Universita Degli Studi di Trieste, 34127 Trieste (IT)
(72) Inventor: DONATI, Ivan, 33039 SEDEGLIANO (IT); MARSICH, Eleonora, 34148 TRIESTE (IT); SACCO, Pasquale, 86097 CHIAUCI (IT); FURLANI, Franco, 34074 MONFALCONE (IT); SCOGNAMIGLIO, Francesca, 34127 TRIESTE (IT)
(74) Representative: Zaccaro, Elisabetta

(57) **Abstract**

The invention concerns a process for the preparation of hydrogels from chitosan oligosaccharide derivatives and boric acid, which allows to obtain homogeneous hydrogels avoiding the formation of precipitates. In another aspect, a hydrogel obtainable from this process is described.

The use of the hydrogels in the biomedical and pharmaceutical fields for applications such as viscosupplementation, as a substitute for the extracellular matrix, and as a drug delivery system are also described.

## Description

### DESCRIPTION OF THE INVENTION

The invention concerns a process for the preparation of hydrogels from chitosan oligosaccharide derivatives and boric acid, which allows to obtain homogeneous hydrogels avoiding the formation of precipitates. In another aspect, the invention describes a hydrogel obtainable by such a process.

The use of the hydrogels in the biomedical and pharmaceutical fields for applications such as viscosupplementation, as a substitute of the extra-cellular matrix and as a device for drug delivery are also described.

### PRIOR ART

Chitosan is a cationic polysaccharide mainly produced by chemical deacetylation of its precursor chitin. Chitosan is composed of two repetitive units in a variable amount, i.e. 2-amino-2-deoxy-glucopyranose (Glucosamine, unit D) and 2-acetamido-2-deoxy-glucopyranose (N-acetyl-glucosamine, unit A) linked by β-1→4 type bonds. The main parameters affecting the solubility of the polymer at neutral pH are the fractions of said units and the molecular weight (MW) of the polymer. In particular, chitosans having medium to high molecular weight and N-acetyl-glucosamine fraction of less than 40%, preferably less than 30%, will precipitate at neutral pH. This means that it is difficult to obtain homogeneous chitosan-based hydrogels at pH close to neutrality instead of non-homogeneous aggregates.

A method to control the formation of homogeneous hydrogels based on chitosan or derivatives thereof by modifying chemical-physical properties, such as temperature and pH, is therefore needed. Hereinafter, the term "precipitate" refers to polymeric chain interactions wherein the solvent (typically water) is released by the polymeric network. Hereinafter, the term "hydrogel" refers to highly hydrated polymeric networks.

WO 2017/001808 reports a method for the production of hydrogels from chitosan in combination with anionic polymers, wherein the chitosan powder and the anionic polymer powder are mixed in solid state. The two powders are then solubilized by using aqueous solvents having a pH at which the anionic polymer is soluble while the chitosan is not. The addition of an acid enables progressive solubilization of chitosan, which can form hydrogels together with the anionic polysaccharide, in acidic conditions. The use of acidic hydrogels in the presence of cells could, however, have suffer from some limitations.

WO 2017/135498 reports a method for the preparation of thermosensitive hydrogels based on chitosan and nucleic acid which are mixed together at room temperature and undergo gelation at human body temperature.

WO 2009/056602 reports a cross-linkable chitosan composition having a fraction of deacetylated units of between 0.3 and 0.75 and a cross-linking agent, which is active in a pH range of between 6 and 10. However, the use of such cross-linking agents often affects the biocompatibility of the final gel.

WO 2013/037965 reports an ionic gel made of chitosan and alginate oligomers. The inventors describe the mixing of a chitosan, having a medium acetylation degree that makes it soluble and mostly uncharged at neutral pH, with alginate oligomers, followed by lowering pH using a proton donor. The final pH of the system is acid. Acid gels could however present some issues related to biomedical aspects such as cell encapsulation in the field of tissue engineering.

Liu et al., International Journal of Pharmaceutics, 414 (2011) 6-15, report a gelation mechanism of chitosan following addition of a "proton consumer". Such polymer and sodium carbonate are initially mixed in acidic condition, and at a low temperature, to obtain a clear solution. Due to temperature increase, the pH of the mixture increases, and the sol-gel transition is observed due to the polymer insolubility at neutral pH that results in the formation of a cloudy, semi-solid aggregate. However, this mechanism reports a controlled "non-homogeneous precipitation" of this polymer rather than a controlled "homogeneous gelation". These examples show strong limitations in terms of controlled gelation of chitosan in order to obtain tridimensional homogeneous structures. Chemical modifications of chitosan have been exploited to improve the solubility thereof at physiological pH. In recent years, several chitosan derivatives were prepared by chemical modification of chitosan polymeric chain. For such modifications, chemical reactions involving amino groups in the glucosamine units are exploited. In particular, the addition of saccharide units (mono- and oligosaccharide units) as side chains allowed to obtain chitosan derivatives that are soluble in water without the need to lower the pH to acidic values.

Recent studies showed that a specific lactose-derived chitosan was biocompatible and able to stimulate the production of cartilage tissue-specific markers, such as type II collagen and aggrecan (Donati et al., Biomaterials, 2005; 26; 987- 998). US 4,424,346 describes the chemical synthesis of chitosan oligosaccharide derivatives and spontaneous formation of gels thereof at a polysaccharide concentration in the range of between 3% and 5%.

EP 2021408 describes the preparation of a homogeneous mixture of chitosan oligosaccharide derivatives and polyanions, such as hyaluronic acid and alginate, without any precipitation of the two components.

EP 2029629 describes the preparation of tridimensional gels made of a mixture of anionic polysaccharides and cationic oligosaccharide chitosan derivatives that are employed as cell encapsulation matrix or pharmaceutical ingredients. The formation of hydrogels is due to the presence of a lyotropic or thermotropic anionic polysaccharide, while the chitosan-derived oligosaccharides only exert a biological role in stimulation of cell proliferation.

In all the reported examples, oligosaccharide chitosan derivatives do not form homogeneous tridimensional matrices.

Boric acid is a reticulating inorganic agent whose reactivity towards diols is pH-dependent. In particular, boric acid was reported to exist as an equilibrium between a less reactive neutral trigonal species and a much more reactive tetra-coordinate anionic form, the latter being more prone to form ester bonds with diols. This tetracoordinate form exists mainly in neutral or alkaline conditions. Boric acid was reported to promote the formation of tridimensional matrices when employed with polymers having hydroxyl groups.

The interaction between oligosaccharide chitosan derivatives and boric acid is known in the literature, and this interaction leads to the formation of transient reticulation in concentrated solutions (Sacco et al., Biomacromolecules; 2017; 18(12); 4206-4213).

The interaction between oligosaccharide chitosan derivatives and boric acid is instantaneous and leads to formation of precipitates, unless a vigorous mechanical stirring is applied. This limits the possibility to use such interaction for the development of homogeneous injectable or *in situ* gelation systems.

The main object of the present invention is therefore to provide homogeneous hydrogels of oligosaccharide chitosan derivatives that do not show the disadvantages due to the formation of precipitates found in previously described hydrogels.

### SUMMARY OF THE INVENTION

It is known that oligosaccharide chitosan derivatives form non-homogeneous precipitates at neutral pH following addition of boric acid unless a vigorous mechanical stirring is employed.

Surprisingly, the inventors have identified a process for the preparation of hydrogels involving the use of homogenizing reagents that allow to obtain homogeneous matrices and visco-elastic materials from oligosaccharide chitosan derivatives. The present invention concerns a process for the preparation of homogeneous hydrogels from oligosaccharide chitosan derivatives, said process having the steps of:
a. solubilize an oligosaccharide chitosan derivative in water, in an amount ranging from 0.5% a 5% w/V;
b. adjust the pH of the solution obtained from step a. to a pH ranging from 4 to 8;
c. prepare a boric acid solution at a concentration ranging from 30 mM to 200 mM and at a pH ranging from 4 to 8;
d. prepare a solution of a homogenizing reagent selected from the group consisting of mannitol, glucose, fructose, sorbitol, tagatose, sorbose, anhydrous 1,4-erythritol, arabinose, ribose, catechol, alizarin red, sialic acid, cis-1,2-cyclopentanediol, glucuronic acid, galactose, xylose, mannose, maltose, and sucrose;
e. add the solution obtained from step d. to the boric acid solution of step c.;
f. add the solution obtained from step e. to the solution obtained from step b. under stirring; and
g. obtain a hydrogel.

In another embodiment, a process for the preparation of a homogeneous hydrogel from oligosaccharide chitosan derivatives is described, said process having the steps of:
a. solubilize an oligosaccharide chitosan derivative in water in an amount ranging from 0.5 to 5% w/V;
b. adjust the pH of the solution obtained from step a. to a pH ranging from 4 to 8;
c. prepare a solution of boric acid at a concentration in a range from 30 mM to 200 mM and at a pH in the range of 4 to 8;
d. prepare a solution of a homogenizing reagent selected from the group consisting of sodium bicarbonate, sodium carbonate, calcium carbonate, and magnesium carbonate;
e. add the solution obtained from step c. to the solution obtained from step b. under stirring;
f. add the solution of step d. to the solution obtained from step e. under stirring; and
g. obtain a hydrogel.

In yet another embodiment, a process for the preparation of a homogeneous hydrogel from oligosaccharide chitosan derivatives is described, said process having the steps of:
a. solubilize an oligosaccharide chitosan derivative in water in an amount ranging from 0.5 to 5% w/V;
b. adjust the pH of the solution obtained from step a. to a pH ranging from 4 to 8;
c. prepare a boric acid solution at a concentration ranging from 30 mM to 200 mM and at a pH ranging from 4 to 8;
d. prepare a solution containing a combination of two or more homogenizing reagents selected from the group consisting of mannitol, glucose, fructose, sorbitol, tagatose, sorbose, anhydrous 1,4-erythritol, arabinose, ribose, catechol, alizarin red, sialic acid, cis-1,2-cyclopentanediol, glucuronic acid, galactose, xylose, mannose, maltose, sucrose, sodium bicarbonate, sodium carbonate, calcium carbonate, and magnesium carbonate;
e. add the solution obtained from step c. to the solution obtained from step b., under stirring;
f. add the solution of step d. to the solution obtained from step e. under stirring; and
g. obtain a hydrogel.

In another aspect, the present invention concerns a process for the preparation of a homogeneous hydrogel from oligosaccharide chitosan derivatives, said process having the steps of:
a. mix, in the solid state, an oligosaccharide chitosan derivative and one or more homogenizing reagents selected from the group consisting of mannitol, glucose, fructose, sorbitol, tagatose, sorbose, anhydrous 1,4-erythritol, arabinose, ribose, catechol, alizarin red, sialic acid, cis-1,2-cyclopentanediol, glucuronic acid, galactose, xylose, mannose, maltose and sucrose, sodium bicarbonate, sodium carbonate, calcium carbonate and magnesium carbonate;
b. prepare a boric acid solution at a concentration ranging from 30 mM to 200 mM and at a pH ranging from 4 to 8;
c. add the solution obtained in step b. to the solid of step a. under stirring or by means of inter-syringe mixing.

In another embodiment, a hydrogel from oligosaccharide chitosan derivatives obtainable by the process disclosed by the present invention is described.

It is further described a hydrogel of oligosaccharide chitosan derivatives obtainable by a process disclosed by the present invention for use as a medicament.

These hydrogels can be used as injectable devices and for the *in situ* formation of hydrogels able to release therapeutic molecules, bioactive agents and/or cells.

In another aspect, a hydrogel from oligosaccharide chitosan derivatives obtainable by another process disclosed by the present invention for use in visco-supplementation is described.

In yet another aspect, the use of a hydrogel from oligosaccharide chitosan derivatives obtainable by the process disclosed by the present invention to promote cellular and tissue growth is described.

It is further described the use of a hydrogel from oligosaccharide chitosan derivatives obtainable according to the process described by the present invention as a substitute of the extracellular matrix, providing a peculiar nonlinear response, and for drug delivery.

In another aspect, the present invention relates to the use of a hydrogel from oligosaccharide chitosan derivatives obtainable by the process disclosed by the present invention in the orthopedic field.

In another aspect, the present invention relates to the use of a hydrogel of oligosaccharide chitosan derivatives obtainable by the process disclosed by the present invention in the ophthalmic field.

In yet another aspect, the present invention relates to the use of a hydrogel from oligosaccharide chitosan derivatives obtainable by the process disclosed by the present invention in the cosmetic field.

### DESCRIPTION OF THE FIGURES

The present invention will be now described in detail and with reference to the attached Figures.

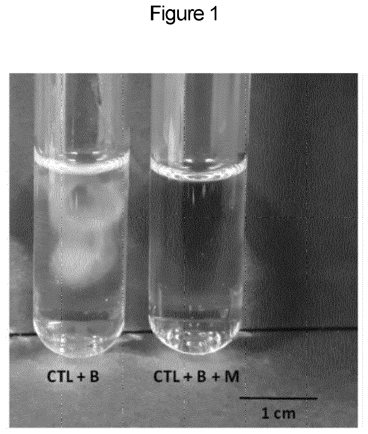

Figure 1. Visual analysis of chitosan oligosaccharide derivatives (CTL) treated with boric acid only (final concentration of 8 mM) (CTL + B) and treated with a mixture of boric acid and mannitol (final concentration of 8 mM 16 mM, respectively) (CTL + B + M).

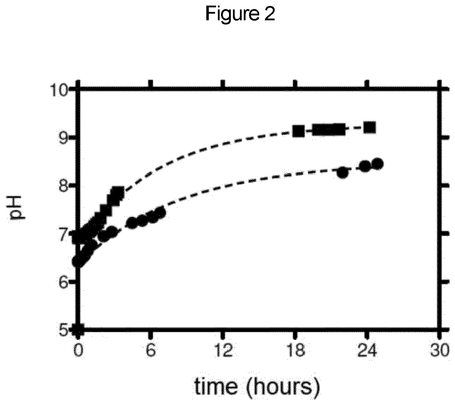

Figure 2. Kinetics of pH of chitosan oligosaccharide derivatives with different amount of sodium bicarbonate (SB): 30 mM SB (circles) and 80 mM SB (squares). SB is added to the chitosan oligosaccharide derivative solutions at pH 5, and the pH changes are recorded using a pH meter electrode. Polymer concentration of 1% w/V, measurements carried out in NaCl 150 mM at room temperature.

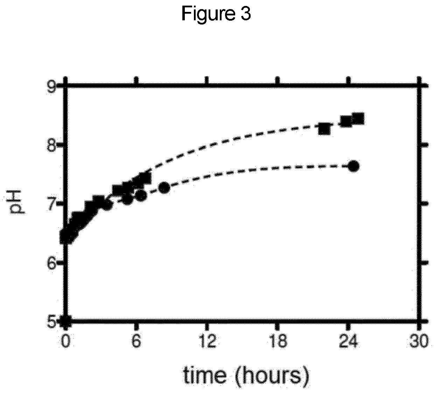

Figure 3. Kinetics of pH changes for the chitosan oligosaccharide (CTL) using sodium bicarbonate (SB) 30 mM. SB is added to the chitosan oligosaccharide derivative solution at pH 5 in the presence (circles) or in the absence (squares) of 10 mM HEPES buffer. pH changes are recorded using a pH meter electrode. Polymer concentration of 1% w/V, measurements carried out in NaCl 150 mM at room temperature.

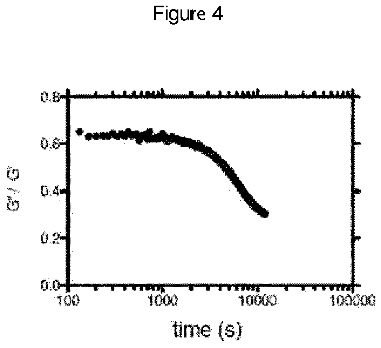

Figure 4. Relationship between viscous and elastic modulus, G" / G', as a function of time for chitosan oligosaccharide derivative (1% w/V) - boric acid (8 mM) with sodium bicarbonate (SB) 30 mM; measurements carried out in NaCl 150 mM at T = 25 °C. Experimental conditions: Time Oscillatory Sweep experiments performed at constant frequency (1 Hz) and applied strain (0.5%) during measurements. The experimental set-up used is the following: titanium plates with a cone-plate geometry (Ø = 60 mm, cone opening angle 1°).

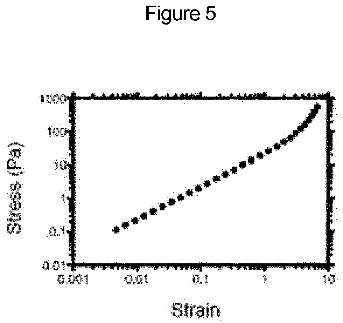

Figure 5. Stress/strain relationship of the homogeneous hydrogel from chitosan oligosaccharide derivative (1% w/V) and boric acid (8 mM) with sodium bicarbonate (SB) 30 mM; measurements carried out in NaCl 150 mM at T = 25 °C. Experimental conditions: Stress-Sweep oscillatory experiments at constant frequency (1 Hz) and stress applied in the range of 0.1 - 10,000 Pa. The experimental set-up used is the following: titanium plates with plate-plate geometry (Ø = 35 mm).

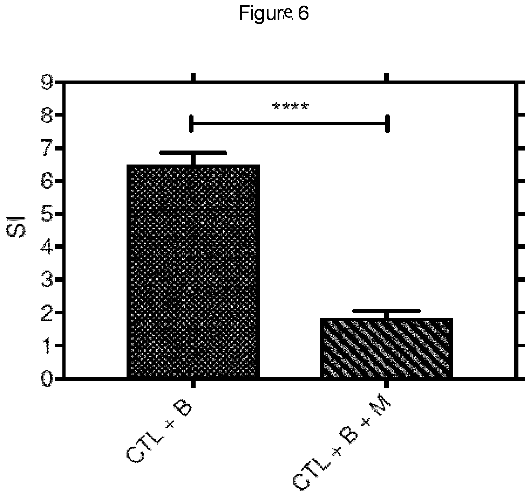

Figure 6. Normalized scattered light intensity (SI) for chitosan oligosaccharide derivative (CTL) treated with boric acid only (final concentration 8 mM) (CTL + B) and treated with a mixture of boric acid and mannitol (final concentration of 8 mM and 16 mM, respectively) (CTL + B + M). Data are reported as scattered light intensity for resulting solution normalized based on scattered light intensity for the polymer without boric acid. Data are reported as mean ± standard deviation (SD) of three measurements. Statistical analyses: student's *t*-test: ****, *p* < 0.0001.

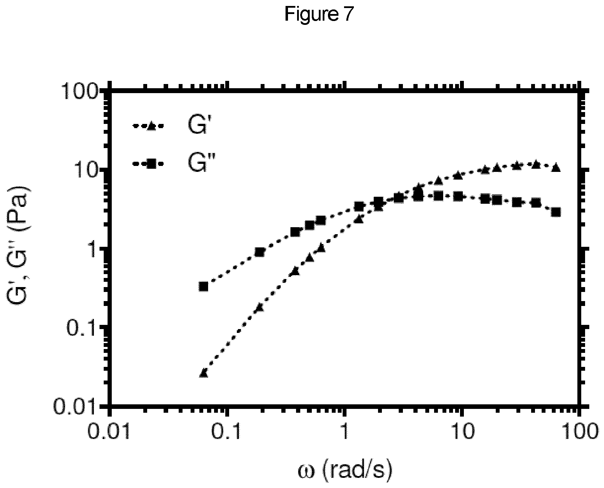

Figure 7. Elastic modulus (G') (triangles) and viscous modulus (G") (squares) as a function of the frequency for a homogeneous hydrogel composed by a chitosan oligosaccharide derivative (1% w/V), boric acid (8 mM) and mannitol (16 mM). Dashed black lines are drawn to guide the eye. Experimental conditions: Frequency Sweep at a constant stress (1 Pa) and applied frequency in the range of 0.1 - 100 Hz (T = 25 °C). The experimental set-up used is the following: titanium plates with a cone-plate geometry (Ø = 60 mm, cone opening angle 1°).

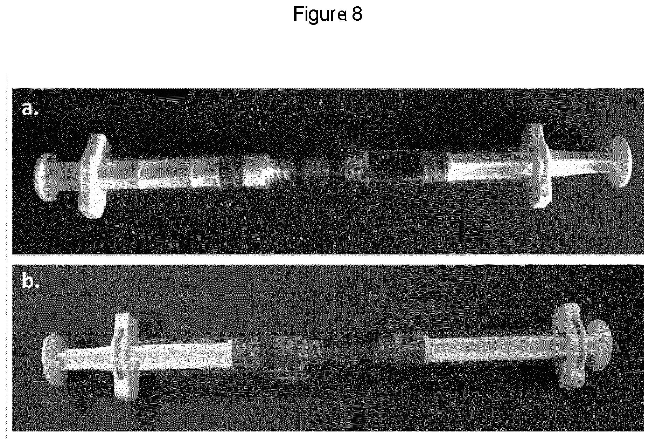

Figure 8. Mixing system used for the production of homogeneous hydrogels. The syringes containing powders (oligosaccharide chitosan derivatives and "proton consumers" reagents - sodium bicarbonate) are connected to a syringe containing the cross-linking agent (boric acid) (a). The homogeneous hydrogels can be obtained by mixing the individual components (b).

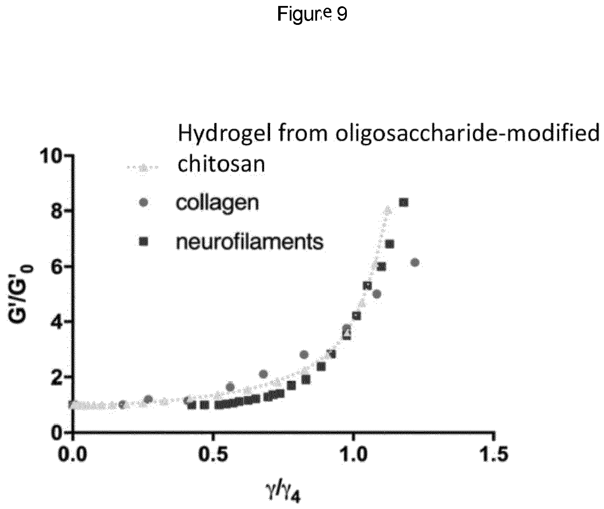

Figure 9. Scaled modulus-strain curves for hydrogels obtained from oligosaccharide-modified chitosan obtained from process according to the present invention as shown in Example 7 (triangle), for collagen (circle) and neurofilaments (square). The latter two were replotted from Strom et al.; Nature; 2005; 435; 191.

### DETAILED DESCRIPTION OF THE INVENTION

The present invention deals with the preparation of homogeneous hydrogels from chitosan oligosaccharide derivatives using boric acid as a transient crosslinking agent.

In particular, by process of the present invention it is possible to overcome the drawback found in previously described hydrogels, i.e. the formation of precipitates. The present invention concerns a process for the preparation of homogeneous hydrogels from oligosaccharide chitosan derivatives, said process having the steps of:
a. solubilize an oligosaccharide chitosan derivative in water, in an amount ranging from 0.5% a 5% w/V;
b. adjust the pH of the solution obtained from step a. to a pH ranging from 4 to 8;
c. prepare a boric acid solution at a concentration ranging from 30 mM to 200 mM and at a pH ranging from 4 to 8;
d. prepare a solution of a homogenizing reagent selected from the group consisting of mannitol, glucose, fructose, sorbitol, tagatose, sorbose, anhydrous 1,4-erythritol, arabinose, ribose, catechol, alizarin red, sialic acid, cis-1,2-cyclopentanediol, glucuronic acid, galactose, xylose, mannose, maltose, and sucrose;
e. add the solution obtained from step d. to the boric acid solution of step c.;
f. add the solution obtained from step e. to the solution obtained from step b. under stirring; and
g. obtain a hydrogel.

In another embodiment, a process for the preparation of a homogeneous hydrogel from oligosaccharide chitosan derivatives is described, said process having the steps of:
a. solubilize an oligosaccharide chitosan derivative in water in an amount ranging from 0.5 to 5% w/V;
b. adjust the pH of the solution obtained from step a. to a pH ranging from 4 to 8;
c. prepare a solution of boric acid at a concentration ranging from 30 mM to 200 mM and at a pH ranging from 4 to 8;
d. prepare a solution of a homogenizing reagent selected from the group consisting of sodium bicarbonate, sodium carbonate, calcium carbonate, and magnesium carbonate;
e. add the solution obtained from step c. to the solution obtained from step b. under stirring;
f. add the solution of step d. to the solution obtained from step e. under stirring; and
g. obtain a hydrogel.

In yet another embodiment, a process for the preparation of a homogeneous hydrogel from oligosaccharide chitosan derivatives is described, said process having the steps of:
a. solubilize an oligosaccharide chitosan derivative in water in an amount ranging from 0.5 to 5% w/V;
b. adjust the pH of the solution obtained from step a. to a pH ranging from 4 to 8;
c. prepare a boric acid solution at a concentration ranging from 30 mM to 200 mM and at a pH ranging from 4 to 8;
d. prepare a solution containing a combination of two or more homogenizing reagents selected from the group consisting of mannitol, glucose, fructose, sorbitol, tagatose, sorbose, anhydrous 1,4-erythritol, arabinose, ribose, catechol, alizarin red, sialic acid, cis-1,2-cyclopentanediol, glucuronic acid, galactose, xylose, mannose, maltose, sucrose, sodium bicarbonate, sodium carbonate, calcium carbonate, and magnesium carbonate;
e. add the solution obtained from step c. to the solution obtained from step b., under stirring;
f. add the solution of step d. to the solution obtained from step e. under stirring; and
g. obtain a hydrogel.

In another aspect, the present invention concerns a process for the preparation of a homogeneous hydrogel from oligosaccharide chitosan derivatives, said process having the steps of:
a. mix, in the solid state, an oligosaccharide chitosan derivative and one or more homogenizing reagents selected from the group consisting of mannitol, glucose, fructose, sorbitol, tagatose, sorbose, anhydrous 1,4-erythritol, arabinose, ribose, catechol, alizarin red, sialic acid, cis-1,2-cyclopentanediol, glucuronic acid, galactose, xylose, mannose, maltose and sucrose, sodium bicarbonate, sodium carbonate, calcium carbonate and magnesium carbonate;
b. prepare a boric acid solution at a concentration ranging from 30 mM to 200 mM and at a pH ranging from 4 to 8;
c. add the solution obtained in step b. to the solid of step a. under stirring or by means of inter-syringe mixing.

In the present invention when the following definition is used:
- "homogenizing reagent" is intended to encompass compounds able to retard crosslinking of the chitosan oligosaccharide derivative, ultimately leading to the formation of colorless and homogeneous hydrogels. Such compounds may be selected from the group consisting of mannitol, glucose, fructose, sorbitol, tagatose, sorbose, anhydrous 1,4-erythritol, arabinose, ribose, catechol, alizarin red, sialic acid, cis-1,2-cyclopentanediol, glucuronic acid, galactose, xylose, mannose, maltose, sucrose, sodium bicarbonate, sodium carbonate, calcium carbonate, and magnesium carbonate
- "chitosan oligosaccharide derivative" is intended to encompass branched polysaccharides in which the oligosaccharides are linked to the chitosan chain. Chitosan oligosaccharide derivatives have a large amount of diol-containing units, typically 1,2- and 1,3-diols. Chitosan oligosaccharide derivatives are preferably branched aldonic or alditolic derivatives of chitosan, where the Glucosamine units in the polymer linear chain can bind, through the functional group NH₂ on carbon C2, mono- or oligosaccharide aldonic or alditolic polyol residues, that can be the same or different from each other, and are represented by the general formula (I) where R can be CH₂ or CO and R1 can be hydrogen or a monosaccharide, preferably selected from the group consisting of galactose, glucose, mannose, N-acetyl-glucosamine and N-acetyl-galactosamine, or an oligosaccharide, and R2 can be OH or NHCOCH₃.

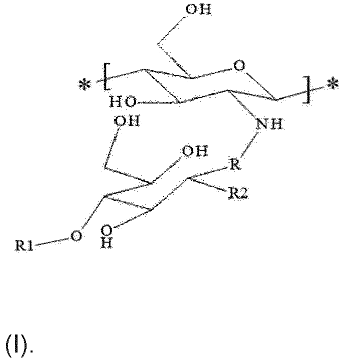

In a preferred embodiment, said chitosan oligosaccharide derivative is obtainable by reductive amination (N-alkylation) with oligosaccharides having 1 to 4 glycosidic units.

In an even more preferred embodiment, the oligosaccharides of said oligosaccharide chitosan derivative are selected from the group consisting of glucose, galactose, lactose, cellobiose, cellotriose, maltose, maltotriose, maltotetraose, chitobiose, chitotriose, melibiose, agarobiose, preferably said oligosaccharide is lactose.

In a further preferred embodiment, the degree of substitution of said oligosaccharide chitosan derivative is in the range of between 5% and 90%, preferably said degree of substitution of chitosan is in the range of between 55 % and 85%, preferably said degree of substitution of chitosan is 60%.

Surprisingly, in the process according to the present invention, it was possible to obtain a controlled interaction between boric acid and diols in the side chains of the polysaccharide and avoid formation of precipitates. In the processes known in the prior art, precipitates correlate with an uncontrolled and inhomogeneous gelation, showing phase separation and loss of solvent (typically water).

Advantageously, in a preferred embodiment of the process according to the present invention, the final concentration of the oligosaccharide chitosan derivative is in the range from 0.5% to 5% w/V, preferably in the range from 0.8% to 2% w/V.

In a preferred embodiment of the process according to the present invention, the final concentration of boric acid is in a range from 1 to 300 mM, preferably in a range from 6 to 50 mM, more preferably in a range from 4 to 20 mM.

In a further preferred embodiment, in the process according to the present invention, the final concentration of the oligosaccharide chitosan derivative is 1% w/V and the final concentration of boric acid is 8 mM.

In a more preferred embodiment, in the process according to the present invention, the homogenizing reagent is selected from the group consisting of sodium bicarbonate, sodium carbonate, calcium carbonate, and magnesium carbonate, preferably said homogenizing reagent is sodium bicarbonate and is added at a concentration ranging from 0.005 M to 5 M, preferably at a concentration ranging from 0.5 M to 2 M.

In a further embodiment of the process according to the present invention, the homogenizing reagent is sodium bicarbonate at a final concentration in the range from 10 to 100 mM, preferably the final concentration of sodium bicarbonate is 30 mM.

The gelation process requires the addition of a homogenizing reagent selected from the group consisting of sodium bicarbonate, sodium carbonate, calcium carbonate and magnesium carbonate, which acts as a "proton consumer".

Sodium bicarbonate (SB) is added in an amount of not less than 1 mM, preferably in a concentration ranging from 30 mM to 100 mM, even more preferably 30 mM. The above reagent will be converted into water and carbon dioxide in acid conditions by means of a bicarbonate buffer system. The loss of carbon dioxide will therefore continuously consume protons with a consequent increase in the pH of the mixture. The slow and constant increase in pH will convert the almost inert form of boric acid into the more reactive one.

The formation of reactive boric acid will therefore promote the homogeneous interaction of the above reagent with chitosan oligosaccharide derivatives by means of the diols in the side chain without inducing precipitation.

The change in pH following the addition of the "proton consumer" was experimentally verified. Surprisingly, the pH change versus time curves show an immediate, albeit moderate, increase in pH, followed by a gradual and significant increase up to 24 hours of incubation (Figure 2).

In a further aspect, in the process according to the present invention, the pH of the solution is adjusted by a buffer reagent selected from the group consisting of acetic acid/sodium acetate (AcOH/AcNa) buffer, citric acid/sodium citrate buffer, 4-(2-hydroxyethyl)piperazine-1-ethanesulfonic acid (HEPES) buffer and phosphate buffered saline (PBS), preferably said buffer reagent is HEPES.

The pH change can optionally be modulated using different amounts of buffer reagents in a typical pH range from 5 to 8, preferably from 6.5 to 7.5. Examples of buffer reagents working in the above pH range include acetic acid/sodium acetate buffer (AcOH/AcNa), citric acid/sodium citrate buffer, 4-(2-hydroxyethyl)piperazine-1-ethanesulfonic acid (HEPES) buffer and phosphate buffered saline (PBS), and it is preferably HEPES buffer (Figure 3).

The change in the viscoelastic properties of the hydrogel composed of oligosaccharide chitosan derivative and boric acid following the addition of sodium bicarbonate was investigated by rheology.

The gelation kinetics shows a first slight decrease in the ratio between the viscous and elastic modulus, G" / G', followed by a sharp decrease, indicating that the progressive gelation of the oligosaccharide chitosan derivative-boric acid mixture becomes considerably stronger over time (Figure 4).

In a further embodiment of the process according to the present invention, a salt is added to the solution obtained from step a., preferably said salt is NaCl at a concentration of 1.5 M.

In a further aspect, in the process according to the present invention, the final concentration of NaCl is in the range from 50 mM to 300 mM, more preferably the final concentration of NaCl is 150 mM.

In a further aspect, in the process according to the present invention, the pH of the solution of steps b., c. and f. is equal to 5.

In a preferred embodiment, the process according to the present invention has the steps of:
a. solubilize 50 mg of an oligosaccharide chitosan derivative in 3 mL of deionized water and add 0.5 mL of 1.5 M NaCl;
b. adjust the pH of the solution obtained from step a. up to pH 5 using 1 M NaOH, add 250 µL of 200 mM HEPES buffer, pH = 5 and adjust the volume of the solution thus obtained to 4.35 mL using deionized water;
c. prepare a boric acid solution at a concentration of 80 mM at pH 5 using 0.1 M NaOH;
d. prepare a 1 M sodium bicarbonate solution in deionized water;
e. add the solution obtained from step c. to the solution obtained from step b. at pH 5 under stirring;
f. add 150 µL of the solution of step d. to the solution obtained from step e., under stirring;
g. obtain a hydrogel.

In a more preferred embodiment, in the process according to the present invention, the homogenizing reagent is selected from the group consisting of mannitol, glucose, fructose, galactitol, lactitol, lactose, sorbitol, tagatose, sorbose, anhydrous 1,4-erythritol, arabinose, ribose, catechol, alizarin red, sialic acid, cis-1,2-cyclopentanediol, glucuronic acid, galactose, xylose, mannose, maltose and sucrose, preferably said homogenizing reagent is mannitol, which acts as a "competing reagent" for the binding of boric acid.

The binding of boric acid to the oligosaccharide chitosan derivative is modulated by the presence of the competitor for the binding of boric acid, which is added to the system. The competitor retards the crosslinking of the oligosaccharide chitosan derivative, leading to the formation of colorless and homogeneous hydrogels. The sol-gel transition time depends on the concentration of the homogenizing reagent used.

In a more preferred embodiment, in the process according to the present invention, the homogenizing reagent is mannitol at a final concentration in the range from 1 to 1,000 mM, preferably the final concentration is in the range from 4 to 40 mM, more preferably the final concentration of mannitol is 16 mM.

The complex between homogenizing reagent and boric acid is able to slowly release boric acid to crosslink chitosan oligosaccharide derivatives with consequent controlled homogeneous gelation without any precipitation.

In a further aspect, in the process according to the present invention, a buffer solution is added to the solution obtained from step a., preferably said buffer solution is added until a concentration range from 1 to 100 mM is obtained, more preferably said buffer solution is phosphate buffered saline (PBS 1x) at a concentration of 10 mM.

In a further embodiment of the process according to the present invention, the pH of the solution in steps b., c. and f. is equal to 7.4.

The addition of boric acid to polymeric solutions at pH 7.4 induces an instantaneous and inhomogeneous gelation of the polymer with the formation of cloudy, viscous and filamentous aggregates (Figure 1). This uncontrolled process is promoted by the very rapid formation of boron esters between the anionic tetracoordinate form of the inorganic component and the lactose diols. Visual observations of oligosaccharide chitosan derivative solutions treated with a mixture of boric acid and mannitol clearly indicate that the use of competitors is fundamental in the formation of a homogeneous (colorless) system (Figure 1).

The homogeneity of the samples obtained using only the crosslinking agent or the boric acid/mannitol mixture was verified by Dynamic Light Scattering (DLS) analysis. In the absence competitors (Figure 6), a marked increase in visible scattered light was detected due to an increase in the polymer mass per unit of volume, thus confirming the presence of large cloudy precipitates. The increase in the intensity of scattered light was limited in the presence of competing reagents, which suggested a marked improvement in the homogeneity of the system.

In a further aspect, in the process according to the present invention, the final concentration of the oligosaccharide chitosan derivative is in the range from 0.5% to 5% w/V, preferably in the range from 0.8% to 2% w/V, even more preferably the final concentration of the oligosaccharide chitosan derivative is 1% w/V.

In a more preferred embodiment, in the process according to the present invention, the final concentration of boric acid is in a range from 1 to 300 mM, preferably in a range from 6 to 50 mM, even more preferably the final boric acid concentration is 8 mM.

In a further aspect, in the process according to the present invention, the final concentration of the homogenizing reagent is in the range from 1 to 1,000 mM, preferably the final concentration of the homogenizing reagent is in the range from 6 to 50 mM, preferably said homogenizing reagent is mannitol at a final concentration of 16 mM.

In a preferred embodiment, the process according to the present invention has the steps of:
a. solubilize an oligosaccharide chitosan derivative in water in an amount ranging from 0.5 to 5% weight/volume and add a phosphate buffered saline (10X PBS) until a concentration of 10 mM is obtained;
b. adjust the pH of the solution obtained from step a. to pH 7.4 by adding NaOH;
c. prepare a boric acid solution at a concentration of 80 mM at pH 7.4;
d. prepare a mannitol solution;
e. add the solution obtained from step d. to the solution of boric acid at pH 7.4 of step c.;
f. adjust the pH of the solution obtained from step e. to pH 7.4 by adding NaOH;
g. add the solution obtained from step f. to the solution obtained from step b. under stirring;
h. obtain a hydrogel.

Chitosan oligosaccharide derivatives are branched polysaccharides in which the oligosaccharides are linked to the chitosan chain. Chitosan oligosaccharide derivatives have a large amount of diol-containing units, typically 1,2- and 1,3-diols. Chitosan oligosaccharide derivatives are preferably branched aldonic or alditolic derivatives of chitosan, where the Glucosamine units in the polymer linear chain can bind, through the functional group NH₂ on carbon C2, mono- or oligosaccharide aldonic or alditolic polyol residues, that can be the same or different from each other, and are represented by the general formula (I) where R can be CH₂ or CO and R1 can be hydrogen or a monosaccharide, preferably selected from the group consisting of galactose, glucose, mannose, N-acetyl-glucosamine and N-acetyl-galactosamine, or an oligosaccharide, and R2 can be OH or NHCOCH₃.

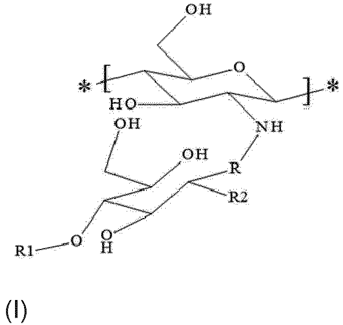

In a preferred embodiment, in the process according to the present invention, said chitosan oligosaccharide derivative is obtainable by reductive amination (N-alkylation) with oligosaccharides having 1 to 4 glycosidic units.

In an even more preferred embodiment, in the process according to the present invention the oligosaccharides of said oligosaccharide chitosan derivative are selected from the group consisting of glucose, galactose, lactose, cellobiose, cellotriose, maltose, maltotriose, maltotetraose, chitobiose, chitotriose, melibiose, agarobiose, preferably said oligosaccharide is lactose.

In a further preferred embodiment, in the process according to the present invention, the degree of substitution of said oligosaccharide chitosan derivative is in the range of between 5% and 90%, preferably said degree of substitution of chitosan is in the range of between 55 % and 85%, preferably said degree of substitution of chitosan is 60%.

In a further embodiment, in the process according to the present invention, chitosan has a molecular weight in the range from 100,000 to 2,000,000, preferably in the range from 200,000 to 800,000 and more preferably from 200,000 to 650,000. The concentration of oligosaccharide chitosan derivative, in the composition with boric acid and/or derivatives thereof, is in the range from 1% to 5% w/V, preferably is in the range from 1% to 3% w/V and more preferably is 1% w/V.

In a further embodiment, a hydrogel from chitosan oligosaccharide derivatives, obtainable according to the process described by the present invention, is disclosed. In a further aspect of the present invention, compositions comprising the hydrogel of chitosan oligosaccharide derivatives, obtainable according to the process described by the present invention, and one or more inorganic salts are further described. Examples of such inorganic salts are sodium chloride, sodium phosphate, sodium dihydrogen phosphate, disodium hydrogen phosphate, potassium chloride, potassium phosphate, potassium dihydrogen phosphate, dipotassium hydrogen phosphate.

The use of oligosaccharide modified chitosan is of fundamental importance in defining the properties of the final hydrogels. In fact, the use of boric acid with chitosan derivatives, such as reported by Abureesh M et al (Int. J. Biol. Macromol.; 2015; 90; 75-80), can lead to hydrogel formation. However, the hydrogels formed are not characterized by non-linear mechanical properties which are of fundamental importance for hydrogels in medical and cosmetic applications. At variance, the hydrogel formed by means of the present technology shows marked non-linear mechanical properties which render it similar to collagen and neurofilaments. This is shown in Figure 9 where the present hydrogel based on oligosaccharide-modified chitosan is compared to collagen and neurofilaments.

In a preferred embodiment, the hydrogel of chitosan oligosaccharide derivatives, obtainable according to the process described by the present invention has a strain strength with non-linear stress-strain ratio up to 600% of the total strain, indicating a strain-hardening behavior (Figure 5). Non-linear rheological behavior is advantageous for tissue engineering applications and these hydrogels could be used in visco-supplementation for osteoarticular pathologies.

Hydrogel strength can possibly be modulated by varying the amount of sodium bicarbonate.

The resulting hydrogels show no syneresis.

In a more preferred embodiment, the hydrogel of chitosan oligosaccharide derivatives, obtainable according to the process described by the present invention, is stable at room temperature for at least 7 days.

The degradation of the hydrogels can possibly be modulated with the temperature and/or the addition of appropriate reagents.

A hydrogel of chitosan oligosaccharide derivatives obtainable according to the process described by the present invention for use for biomedical applications, is further described.

The hydrogels obtainable according to the present invention have a potential application in the formation of biomaterials that can be used in the biomedical field, especially for the encapsulation of cells. Homogeneous systems are to be preferred in the development of biomaterials to uniformly distribute the strains to which they are subjected and to promote efficient cell infiltration and consequent colonization, when the material is intended for use in tissue engineering purposes.

The change in viscoelastic properties of a hydrogel composed of the oligosaccharide chitosan derivative and boric acid in the presence of a homogenizing reagent was analyzed by rheology (Figure 7).

The mechanical performance of the resulting homogeneous hydrogels can be modulated by varying the amount of the homogenizing reagent used.

In another aspect, a hydrogel of chitosan oligosaccharide derivatives obtainable according to the process described by the present invention for use in viscosupplementation is described.

In still another aspect, the use of a hydrogel of chitosan oligosaccharide derivatives, obtainable according to the process described by the present invention as a substitute for the extracellular matrix giving a peculiar non-linear response, for the encapsulation of cells and to promote cellular and tissue growth are described. The use of a hydrogel of chitosan oligosaccharide derivatives obtainable according to the process described by the present invention for drug delivery is further described.

The combination of the homogenizing reagent solution and the solution comprising the oligosaccharide chitosan derivative can be obtained both in solution and in the solid state (powders). In the latter case, the rehydration of the solid mixture can be achieved through simple mixing with an aqueous solution by using two or more syringes connected with a Luer-Lock system. A composition can be obtained by mixing the homogenizing reagents and the oligosaccharide chitosan derivative both in solution and in the solid state.

Using the Luer-Lock system of interconnected syringes, the formation of hydrogels is allowed by means of consecutive transfers of the individual reagents. Said reagents can be used in the form of a powder or as a solution. At least one aqueous solution is required for their mixing. The mixing of the reagents allows the formation of homogeneous hydrogels after consecutive transfers in an estimated time of minutes. Continuous transfers between interconnected syringes promote the rehydration of the powder components while the hydrogel is formed (Figure 8).

In all reported compositions, the present invention leads to homogeneous hydrogels of chitosan oligosaccharide derivatives at neutral or slightly acidic or slightly alkaline pH that show self-healing properties.

The present invention provides simple methodologies to prepare homogeneous hydrogels for use in viscosupplementation, in drug delivery, in tissue engineering and for cells encapsulation.

The present invention relates to the use of a homogenizing reagent to bind boric acid and/or use of a proton-consuming reagent to finely modulate the formation of homogeneous hydrogels composed of boric acid and chitosan oligosaccharide derivatives to be used as injectable systems or for tissue engineering.

The second object of the present invention is the exploitation of competition with other binding molecules or pH-induced gelation for the *in situ* release of therapeutic molecules, bioactive agents and/or cells.

In another aspect, the present invention relates to the use of a hydrogel of chitosan oligosaccharide derivatives, obtainable according to the process described by the present invention, in the orthopedic field.

In a further aspect, the present invention relates to the use of a hydrogel of chitosan oligosaccharide derivatives, obtainable according to the process described by the present invention, in the ophthalmic field.

In still another aspect, the present invention relates to the use of a hydrogel of chitosan oligosaccharide derivatives, obtainable according to the process described by the present invention, in the cosmetic field.

Examples of embodiments of the present invention are provided below for illustrative purposes.

### EXAMPLES

### Example 1. Formation of homogeneous hydrogels containing chitosan oligosaccharide derivatives and boric acid using 30 mM sodium bicarbonate as homogenizing reagent.

50 mg of the chitosan oligosaccharide derivative are weighed and solubilized using 3 mL of deionized water. After complete solubilization, 0.5 mL of 1.5 M NaCl are added. The pH of the solution is then adjusted to 5 using 1 M NaOH. The volume is then adjusted to 4.35 mL using deionized water. The boric acid solution is prepared separately at a concentration of 80 mM and the pH adjusted to 5 using 0.1 M NaOH. The boric acid solution (0.5 mL) is then added to the oligosaccharide chitosan derivative solution under stirring. A 1 M sodium bicarbonate solution is prepared just before use using deionized water as a solvent. Then, 150 µL of sodium bicarbonate are added to the oligosaccharide chitosan derivative solution under stirring in order to reach a final volume of 5 mL. After mixing for about 10 s, the mixture composed of oligosaccharide chitosan derivative and boric acid (2 mL) was dispensed into 6-well plates (Ø ∼ 3.5 cm) by means of a syringe and let stand for 24 hours. The final experimental conditions are: [oligosaccharide chitosan derivative] = 1% w/V, [boric acid] = 8 mM, [NaCl] = 150 mM, and [sodium bicarbonate] = 30 mM. The whole process is performed at room temperature. The final pH of the mixture after 24 hours of incubation is 8.4.

The resulting hydrogel is clear, shows no syneresis and is stable at room temperature. This hydrogel has the rheological properties shown in Figure 5.

### Example 2. Formation of homogeneous hydrogels containing chitosan oligosaccharide derivatives and boric acid using 100 mM sodium bicarbonate as homogenizing reagent.

50 mg of the chitosan oligosaccharide derivative are weighed and solubilized using 3 mL of deionized water. After complete solubilization, 0.5 mL of 1.5 M NaCl are added. The pH of the solution is then adjusted to 5 using 1 M NaOH. The volume is then adjusted to 4 mL using water. The boric acid solution is prepared separately at a concentration of 80 mM and the pH adjusted to 5 using 0.1 M NaOH. The boric acid solution (0.5 mL) is then added to the oligosaccharide chitosan derivative solution under stirring. A 1 M sodium bicarbonate solution is prepared just before use by using deionized water as a solvent. Then, 500 µL of sodium bicarbonate were added to the oligosaccharide chitosan derivative solution under stirring to obtain a final volume of 5 mL. After mixing for about 10 s, the mixture composed of oligosaccharide chitosan derivative and boric acid (2 mL) was dispensed into 6-well plates (Ø ∼ 3.5 cm) by means of a syringe and let stand for 24 hours. The final experimental conditions are: [oligosaccharide chitosan derivative] = 1% w/V, [boric acid] = 8 mM, [NaCl] = 150 mM, and [sodium bicarbonate] = 100 mM. The whole process is performed at room temperature. The final pH of the mixture after 24 hours of incubation is 8.9.

The resulting hydrogel is clear, shows no syneresis and is stable at room temperature. This hydrogel has poorer rheological mechanical characteristics than the hydrogel of the previous example (Example 1).

### Example 3. Formation of homogeneous hydrogels containing chitosan oligosaccharide derivatives and boric acid using 30 mM sodium bicarbonate as homogenizing reagent in the presence of 10 mM HEPES buffer.

50 mg of the oligosaccharide chitosan derivative are weighed and solubilized using 3 mL of deionized water. After complete solubilization, 0.5 mL of 1.5 M NaCl are added. The pH of the solution is then adjusted to 5 using 1 M NaOH. 250 µL of 200 mM HEPES buffer, pH = 5, are added. The volume is then adjusted to 4.35 mL using deionized water. The boric acid solution is separately prepared at a concentration of 80 mM and the pH adjusted to 5 using 0.1 M NaOH. The boric acid solution (0.5 mL) is then added to the oligosaccharide chitosan derivative solution under stirring. A 1 M sodium bicarbonate solution is prepared just before use using deionized water as solvent. Then, 150 µL of sodium bicarbonate were added to the oligosaccharide chitosan derivative solution under stirring in order to have a final volume of 5 mL. After mixing for about 10 s, the mixture composed of oligosaccharide chitosan derivative and boric acid (2 mL) was dispensed in 6-well plates (Ø ∼ 3.5 cm) by means of a syringe and let stand for 24 hours. The final experimental conditions are: [oligosaccharide chitosan derivative] = 1% w/V, [boric acid] = 8 mM, [NaCl] = 150 mM, [HEPES] = 10 mM, and [sodium bicarbonate] = 30 mM. The whole process is performed at room temperature. The final pH of the mixture after 24 hours of incubation is 7.6. The pH is monitored by pH meter and follows the trend shown in Figure 3.

### Example 4. Evaluation of the gelation kinetics of the mixture composed of oligosaccharide chitosan derivative and boric acid using 30 mM sodium bicarbonate as homogenizing reagent.

50 mg of the oligosaccharide chitosan derivative are weighed and solubilized using 3 mL of deionized water. After complete solubilization, 0.5 mL of 1.5 M NaCl are added. The pH of the solution is then adjusted to 5 using 1 M NaOH. The volume is then adjusted to 4.35 mL using deionized water. The boric acid solution is prepared separately at a concentration of 80 mM and the pH adjusted to 5 using 0.1 M NaOH. The boric acid solution (0.5 mL) is then added to the oligosaccharide chitosan derivative solution under stirring. A 1 M sodium bicarbonate is prepared just before use by using deionized water as a solvent. Then, 150 µL of sodium bicarbonate were added to the mixture containing oligosaccharide chitosan derivative and boric acid in order to have a final volume of 5 mL. The whole process is performed at room temperature. The final experimental conditions are: [oligosaccharide chitosan derivative] = 1% w/V, [boric acid] = 8 mM, [NaCl] = 150 mM and [sodium bicarbonate] = 30 mM. After mixing for about 10 s, the mixture (about 2.5 mL) was poured on the rheometer plate by means of a syringe and Time-Sweep measurements were performed. The experimental conditions were constant frequency (1 Hz) and applied stress (0.5%) during the measurement. Rheological measurements were performed using a HAAKE MARS III (Thermo Scientific) rheometer, T = 25 °C. The experimental setup used was titanium plates with a cone-plate geometry (Ø = 60 mm, cone opening angle 1°), gap 0.051 mm. The gelation process, monitored by the rheometer, is shown in Figure 4.

### Example 5. Stress-Sweep tests performed on mixtures of chitosan oligosaccharide derivatives and boric acid, using 30 mM sodium bicarbonate as homogenizing reagent.

50 mg of chitosan oligosaccharide derivative are weighed and solubilized in 3 mL of deionized water. After complete solubilization, 0.5 mL of 1.5 M NaCl are added. The pH of the solution is then adjusted to 5 using 1 M NaOH. The volume is then adjusted to 4.35 mL using water. Boric acid is prepared separately at a concentration of 80 mM and the pH adjusted to 5 using 0.1 M NaOH. Under magnetic stirring, the boric acid solution (0.5 mL) is then added to the oligosaccharide chitosan derivative solution. A sodium bicarbonate solution (1 M) is prepared just before the injection using deionized water as solvent. Then, 150 µL of sodium bicarbonate were injected into the oligosaccharide chitosan derivative solution under stirring in order to have a final volume of 5 mL. The whole process is performed at room temperature. The final experimental conditions are: [oligosaccharide chitosan derivative] = 1% w/V, [boric acid] = 8 mM, [NaCl] = 150 mM and [sodium bicarbonate] = 30 mM. After mixing for about 10 s, the mixture composed of oligosaccharide chitosan derivative and boric acid (2 mL) was dispensed into 6-well plates (Ø ∼ 3.5 cm) by means of a syringe and let stand for 24 hours. At the end of the incubation, the hydrogel was placed on the rheometer and oscillatory Stress-Sweep measurements were performed. The experimental conditions are constant frequency (1 Hz) and applied stress (0.1 -10,000 Pa). Rheological measurements are performed using a HAAKE MARS III rheometer (Thermo Scientific) which operates at T = 25 °C. The experimental setting used is titanium plates with flat/flat geometry (Ø = 35 mm), 1 mm gap. The mechanical characteristics of the hydrogel are shown in Figure 5.

### Example 6. Formation of homogeneous hydrogels based on chitosan oligosaccharide derivatives and boric acid using 30 mM sodium bicarbonate as homogenizing reagent.

150 mg of an oligosaccharide chitosan derivative are weighed and solubilized using 3 mL of deionized water. After complete solubilization, 0.5 mL of 1.5 M NaCl are added. The pH of the solution is then adjusted to 5 using 1 M NaOH. The volume is then adjusted to 4.35 mL using water. Boric acid solution is prepared separately at a concentration of 80 mM and the pH adjusted to 5 using 0.1 M NaOH. The boric acid solution (0.5 mL) is then added to the solution containing the oligosaccharide chitosan derivative under stirring. A 1 M sodium bicarbonate solution is prepared just before use by using deionized water as a solvent. Then, 150 µL of sodium bicarbonate were added to the oligosaccharide chitosan derivative solution under stirring in order to have a final volume of 5 mL. After mixing for about 10 s, the mixture of the oligosaccharide chitosan derivative/boric acid (2 mL) was dispensed into 6-well plates (Ø ∼ 3.5 cm) by means of a syringe and let stand for 24 hours. The final experimental conditions are: [oligosaccharide chitosan derivative] = 3% w/V, [boric acid] = 8 mM, [NaCl] = 150 mM and [sodium bicarbonate] = 30 mM. The whole process is performed at room temperature.

### Example 7. Formation of homogeneous hydrogels based on chitosan oligosaccharide derivatives and boric acid using 16 mM mannitol as homogenizing reagent and 8 mM boric acid as crosslinking agent.

50 mg of an oligosaccharide chitosan derivative are weighed and solubilized using 3.5 mL of deionized water. After complete solubilization, 0.5 mL of 10X PBS (phosphate buffered saline) are added. The pH of the solution is then adjusted to 7.4 using 1 M NaOH. The volume is then adjusted to 4.5 mL using deionized water. The mixture of boric acid and mannitol is prepared separately at a concentration of 80 mM for boric acid and 160 mM for mannitol, using PBS as a solvent. The pH of the solution is then adjusted to 7.4 using 1 M NaOH.

The mixture of boric acid and mannitol (0.5 mL) is then added to the oligosaccharide chitosan derivative solution under stirring. The final experimental conditions are: [oligosaccharide chitosan derivative] = 1% w/V, [boric acid] = 8 mM, [mannitol] = 16 mM, PBS 1X and pH 7.4. The whole process is performed at room temperature. The resulting hydrogel is clear (Figure 1), shows no syneresis and is stable at room temperature. The mechanical characteristics of the hydrogel are shown in Figure 7.

### Example 8. Formation of hydrogels based on an oligosaccharide chitosan derivative and boric acid using 8 mM mannitol as homogenizing reagent and 8 mM boric acid as crosslinking agent.

50 mg of an oligosaccharide chitosan derivative are weighed and solubilized using 3.5 mL of deionized water. After complete solubilization, 0.5 mL of 10X PBS (phosphate buffered saline) are added. The pH of the solution is then adjusted to 7.4 using 1 M NaOH. The volume is then adjusted to 4.5 mL using deionized water. The mixture of boric acid and mannitol is prepared separately at a concentration of 80 mM for boric acid and 80 mM for mannitol, using PBS as a solvent. The pH of the solution is brought to 7.4 using 1 M NaOH. The mixture of boric acid and mannitol (0.5 mL) is then added to the solution containing the oligosaccharide chitosan derivative, under magnetic stirring. The final experimental conditions are: [oligosaccharide chitosan derivative] = 1% w/V, [boric acid] = 8 mM, [mannitol] = 8 mM, PBS 1X and pH 7.4. The whole process is performed at room temperature. The resulting hydrogel is clear, shows no syneresis and is stable at room temperature. The mechanical characteristics of the hydrogel are similar to those shown in Figure 7.

### Example 9. Formation of hydrogels based on an oligosaccharide chitosan derivative and boric acid using 8 mM mannitol as homogenizing reagent and 4 mM boric acid as crosslinking agent.

50 mg of an oligosaccharide chitosan derivative are weighed and solubilized using 3.5 mL of deionized water. After complete solubilization, 0.5 mL of 10X PBS (phosphate buffered saline) are added. The pH of the solution is then adjusted to 7.4 using 1 M NaOH. The volume is then adjusted to 4.5 mL using deionized water. The mixture of boric acid and mannitol is separately prepared at a concentration of 40 mM for boric acid and 80 mM for mannitol using PBS as a solvent. The pH is adjusted to 7.4 using 1M NaOH. The mixture of boric acid and mannitol (0.5 mL) is then added to the solution containing the oligosaccharide chitosan derivative under magnetic stirring. The final experimental conditions are: [oligosaccharide chitosan derivative] = 1% w/V, [boric acid] = 4 mM, [mannitol] = 8 mM, PBS 1X and pH 7.4. The whole process is performed at room temperature. The resulting hydrogel is clear, shows no syneresis and is stable at room temperature. The mechanical characteristics of the hydrogel are similar to those shown in Figure 7.

### Example 10. Formation of hydrogels based on an oligosaccharide chitosan derivative and boric acid using 8 mM mannitol as homogenizing reagent and 8 mM boric acid as crosslinking agent.

150 mg of an oligosaccharide chitosan derivative are weighed and solubilized using 3.5 mL of deionized water. After complete solubilization, 0.5 mL of 10X PBS (phosphate buffered saline) are added. The pH of the solution is then adjusted to 7.4 using 1 M NaOH. The volume is then adjusted to 4.5 mL using deionized water. The mixture of boric acid and mannitol is separately prepared at a concentration of 80 mM for boric acid and 80 mM for mannitol using PBS as a solvent. The pH is adjusted to 7.4 using 1M NaOH. The mixture of boric acid and mannitol (0.5 mL) is then added to the oligosaccharide chitosan derivative solution under stirring. The final experimental conditions are: [oligosaccharide chitosan derivative] = 3% w/V, [boric acid] = 8 mM, [mannitol] = 8 mM, PBS 1X and pH 7.4. The whole process is performed at room temperature. The resulting hydrogel is clear, shows no syneresis and is stable at room temperature. The mechanical characteristics of the hydrogel are similar to those shown in Figure 7.

### Example 11. Stress-Sweep test on hydrogel based on an oligosaccharide chitosan derivative and boric acid using 16 mM mannitol as homogenizing reagent and 8 mM boric acid as crosslinking agent.

50 mg of an oligosaccharide chitosan derivative are weighed and solubilized using 3.5 mL of deionized water. After complete solubilization, 0.5 mL of 10X PBS (phosphate buffered saline) are added. The pH of the solution is then adjusted to 7.4 using 1 M NaOH. The volume is then adjusted to 4.5 mL using deionized water. The mixture of boric acid and mannitol is separately prepared at a concentration of 80 mM for boric acid and 160 mM for mannitol using PBS as solvent and the pH is adjusted to 7.4 using 1M NaOH. The mixture of boric acid and mannitol (0.5 mL) is then added to the solution containing the oligosaccharide chitosan derivative, under magnetic stirring. The final experimental conditions are: [oligosaccharide chitosan derivative] = 1% w/V, [boric acid] = 8 mM, [mannitol] = 16 mM, PBS 1X and pH 7.4. The whole process is performed at room temperature.

After mixing for about 10 s, the samples (about 2 mL) were poured on the rheometer plates by means of a syringe and the Stress-Sweep oscillatory measurements were performed. The experimental conditions are constant frequency (1 Hz) and applied stress (0.1 - 10,000 Pa). Rheological measurements are performed using a HAAKE MARS III rheometer (Thermo Scientific) which operates at T = 25°C. The experimental setting used is titanium plates with a cone-plate geometry (Ø = 60 mm, cone opening angle 1°), gap 0.051 mm.

### Example 12. Formation of hydrogels based on chitosan oligosaccharide derivatives and boric acid by means of a mixing system, using 30 mM sodium bicarbonate as homogenizing reagent.

60 mg of oligosaccharide chitosan derivative and 8 mg of sodium bicarbonate were weighed and added to a Luer-Lock syringe (Figure 8). An 8 mM boric acid solution at pH 5.2 in 1X PBS (3 mL) was placed in the second Luer-Lock syringe. The two syringes were connected via the Luer-Lock system and the solubilization of the powders was achieved by means of consecutive transfers of the aqueous solution. The final concentration of chitosan oligosaccharide derivatives and sodium bicarbonate in the solution were 2% w/V and 30 mM, respectively. The pH of the solution was equal to 6.22 after standing for 24 hours. The whole process was performed at room temperature.

The resulting hydrogel is clear, shows no syneresis and is stable at room temperature.

### Example 13. Formation of hydrogels based on an oligosaccharide chitosan derivative and boric acid using 16 mM mannitol as homogenizing reagent, 30 mM sodium bicarbonate, and 8 mM boric acid as crosslinking agent.

50 mg of oligosaccharide chitosan derivative are weighed and solubilized in 3 mL of deionized water. After complete solubilization, 0.5 mL of 1.5 M NaCl are added. The pH of the solution is then adjusted to 5 using 1 M NaOH. The volume is then adjusted to 4.35 mL using deionized water. The mixture of boric acid and mannitol is separately prepared at a concentration of 80 mM for boric acid and 160 mM for mannitol, and the pH adjusted to 5 using 0.1 M NaOH. The mixture of boric acid and mannitol (0.5 mL) is then added to the oligosaccharide chitosan derivative solution under magnetic stirring. A sodium bicarbonate (1 M) is prepared just before use by using deionized water as a solvent. Then, 150 µL of sodium bicarbonate were injected into the oligosaccharide chitosan derivative solution under stirring in order to have a final volume of 5 mL. The whole process is performed at room temperature. The final experimental conditions are: [oligosaccharide chitosan derivative] = 1% w/V, [boric acid] = 8 mM, [mannitol] = 16 mM, [NaCl] = 150 mM, and [sodium bicarbonate] = 30 mM. The whole process is performed at room temperature.

The resulting hydrogel is colorless, shows no syneresis and is stable at room temperature.

From the detailed description and from the Examples reported above, the advantages achieved by the method of the present invention are apparent. In particular, this method has proved to be surprisingly and advantageously suitable for the preparation of homogeneous hydrogels. At the same time, this method, being fast and extremely easy to perform, can be conveniently performed in any type of laboratory.

## Claims

1. A process for the preparation of a homogeneous hydrogel from chitosan oligosaccharide derivatives, said process having the steps of:
a. solubilize an oligosaccharide chitosan derivative in water in an amount ranging from 0.5 to 5% w/V;
b. adjust the pH of the solution obtained from step a. to a pH ranging from 4 to 8;
c. prepare a boric acid solution at a concentration ranging from 30 mM to 200 mM and at a pH ranging from 4 to 8;
d. prepare a solution of mannitol as_a homogenizing reagent;
e. add the solution obtained from step d. to the solution of step c.;
f. add the boric acid solution obtained from step e. to the solution obtained from step b. under stirring; and
g. obtain a hydrogel.

2. A process for the preparation of a homogeneous hydrogel from chitosan oligosaccharide derivatives, said process having the steps of:
a. solubilize an oligosaccharide chitosan derivative in water in an amount ranging from 0.5 to 5% w/V;
b. adjust the pH of the solution obtained from step a. to a pH ranging from 4 to 8;
c. prepare a boric acid solution at a concentration ranging from 30 mM to 200 mM and at a pH ranging from 4 to 8;
d. prepare a solution of a homogenizing reagent selected from the group consisting of sodium bicarbonate, sodium carbonate, calcium carbonate, and magnesium carbonate;
e. add the solution obtained from step c. to the solution obtained from step b. under stirring;
f. add the solution of step d. to the solution obtained from step e. under stirring; and
g. obtain a hydrogel.

3. A process for the preparation of a homogeneous hydrogel from chitosan oligosaccharide derivatives, said process having the steps of:
a. solubilize an oligosaccharide chitosan derivative in water in an amount ranging from 0.5 to 5% w/V;
b. adjust the pH of the solution obtained from step a. to a pH ranging from 4 to 8;
c. prepare a boric acid solution at a concentration ranging from 30 mM to 200 mM and at a pH ranging from 4 to 8;
d. prepare a solution containing mannitol;
e. add the solution obtained from step c. to the solution obtained from step b. under stirring;
f. add the solution of step d. to the solution obtained from step e. under stirring; and
g. obtain a hydrogel.

4. A process for the preparation of a homogeneous hydrogel from chitosan oligosaccharide derivatives, said process having the steps of:
a. mix, in the solid state, an oligosaccharide chitosan derivative and mannitol as a homogenizing reagent;
b. prepare a boric acid solution at a concentration ranging from 30 mM to 200 mM and at a pH ranging from 4 to 8;
c. add the solution obtained in step b. to the solid of step a. under stirring or by means of inter-syringe mixing.

5. The process according to any one of claims 1 to 4, wherein the homogenizing reagent is mannitol at a final concentration in the range from 1 to 1,000 mM, preferably the final concentration of mannitol being 16 mM.

6. The process according to claim 5, wherein a buffer solution is added to the solution obtained from step a., preferably said buffer solution being added until a concentration ranging from 1 to 100 mM is obtained, more preferably said buffer solution being phosphate buffered saline (PBS 1x) at a concentration of 10 mM.

7. The process according to any one of claims 1 to 6, wherein the pH of the solution of steps b., c. and f. are equal to pH 7.4.

8. The process according to any one of claims 1 to 7, wherein the final concentration of the oligosaccharide chitosan derivative is in the range from 0.5% to 5% w/V, preferably in the range from 0.8% to 2% w/V, even more preferably the final concentration of the chitosan oligosaccharide derivative being 1% w/V.

9. The method according to any one of claims 1 to 8, wherein the final boric acid concentration is in a range from 1 to 300 mM, preferably in a range from 6 to 50 mM, even more preferably the final boric acid concentration being 8 mM.

10. The process according to any one of claims 1 to 9, wherein the final concentration of the homogenizing reagent is in the range from 1 to 1,000 mM, preferably the final concentration of the homogenizing reagent being in the range from 6 to 50 mM, preferably said homogenizing reagent being mannitol at a final concentration of 16 mM.

11. The process according to any one of claims 1 to 10, said process having the steps of:
a. solubilize an oligosaccharide chitosan derivative in water in an amount ranging from 0.5 to 5% weight/volume and add a phosphate buffered saline (10X PBS) until a final concentration equal to 10 mM is obtained;
b. adjust the pH of the solution obtained from step a. to pH 7.4, by adding NaOH;
c. prepare a boric acid solution at a concentration of 80 mM at pH 7.4;
d. prepare a mannitol solution;
e. add the solution obtained from step d. to the solution of boric acid at pH 7.4 from step c.;
f. adjust the pH of the solution obtained from step e. to pH 7.4, by adding NaOH;
g. add the solution obtained from step f. to the solution obtained from step b. under stirring;
h. obtain a hydrogel.

12. A hydrogel of chitosan oligosaccharide derivatives, obtainable according to any one of claims 1 to 11, having a strain strength with non-linear stress-strain ratio up to 600%, wherein said hydrogel is stable at room temperature for at least 7 days.

13. A hydrogel of chitosan oligosaccharide derivatives according to claim 12, for use as a medicament.

14. A hydrogel of chitosan oligosaccharide derivatives according to claim 12, for use in viscosupplementation.

15. Use of a hydrogel of oligosaccharide chitosan derivatives according to claim 12, to promote cell and tissue growth.

16. A hydrogel of chitosan oligosaccharide derivatives according to claim 12, for use in the orthopedic field.

17. A hydrogel of chitosan oligosaccharide derivatives according to claim 12, for use in the ophthalmic field.
